# EUROPÄISCHE PATENTSCHRIFT

(11) **EP 0 798 693 B1**
(45) Veröffentlichungstag und Bekanntmachung des Hinweises auf die Patenterteilung: **05.06.2002**
(21) Anmeldenummer: 97103811.2
(22) Anmeldetag: 07.03.1997
(51) Int. Cl.: G10K 9/12

(54) **Elektromagnetische Stosswellenquelle**
Electromagnetic shockwave generator
Générateur électromagnétique d'ondes de choque

(30) Priorität: 27.03.1996 DE 19612061
(43) Veröffentlichungstag der Anmeldung: 01.10.1997
(73) Patentinhaber: Dornier Medizintechnik GmbH, 82231 Wessling (DE)
(72) Erfinder: Eizenhöfer, Harald, Dipl.-Phys., 82229 Seefeld (DE)
(74) Vertreter: Grünecker, Kinkeldey, Stockmair & Schwanhäusser Anwaltssozietät

(56) Entgegenhaltungen:
- DE-C- 4 242 131
- US-A- 4 905 675
- US-A- 5 283 836

## Beschreibung

Die vorliegende Erfindung betrifft eine elektromagnetische Stoßwellenquelle, deren die Stoßwellen erzeugender Teil eine Membran aus elektrisch leitendem Material aufweist, die auf der einen Seite mit dem akustischen Ausbreitungsmedium in Berührung steht und die auf ihrer anderen Seite über einen Isolator mit einer Flachspule in Verbindung steht, wobei die Membran unter mechanischer Vorspannung steht.

Derartige Stoßwellenquellen werden für unterschiedlichste Zwecke verwendet, z. B. in der Medizin, um im Körper eines Patienten befindliche Konkremente nicht invasiv zu zertrümmern oder pathologische Gewebeveränderungen ebenfalls nicht invasiv zu behandeln, wobei im ersten Fall positive Druckimpulse, d. h. Überdruckimpulse eingesetzt werden. Außerdem können derartige Stoßwellenquellen beispielsweise in der Werkstoffprüfung eingesetzt werden, um diese mit Druckimpulsen zu beaufschlagen.

Die Stoßwellenquelle wird dabei in geeigneter Weise mit dem jeweils zu beschallenden Objekt akustisch gekoppelt, so daß die in dem akustischen Ausbreitungsmedium erzeugten Druckimpulse in das Objekt eingeleitet werden können. Wesentliches Bauteil einer derartigen Stoßwellenquelle ist dabei der die Stoßwellen erzeugende Teil, der üblicherweise aus einer elektrisch leitfähigen Membran und einer dieser gegenüberliegenden Flachspule besteht. Die Stoßwellen werden dadurch erzeugt, daß die Flachspule an eine Hochspannungsversorgung angeschlossen ist, die z. B. einen auf mehrere kV aufgeladenen Kondensator aufweist, der stoßartig entladen wird, wobei der die Flachspule durchfließende Entladungsstrom in kurzer Zeit ein Magnetfeld aufbaut, das wiederum in der Membran einen Strom erzeugt, der demjenigen in der Flachspule entgegengesetzt ist, so daß ein magnetisches Gegenfeld aufgebaut wird, unter dessen Wirkung die Membran schlagartig von der Flachspule wegbewegt wird. Die dabei auftretende Stoßwelle wird über ein entsprechendes akustisches Ausbreitungsmedium, z. B. entgastes Wasser, deren Bestimmungszweck zugeführt, z. B. dem Körper eines Patienten.

Um einen hohen Wirkungsgrad, d. h. eine möglichst weitgehende Umwandlung der aufgewendeten elektrischen Energie in akustische Stoßwellenenergie zu erzielen, ist es erforderlich, die Membran möglichst nahe an der Flachspule anliegen zu lassen. Ferner muß gewährleistet sein, daß die Membran während des Zeitraums zwischen zwei aufeinanderfolgenden Stoßwellen in ihre Ausgangslage zurückkehrt.

Bei den herkömmlichen elektromagnetischen Stoßwellenquellen ist es daher üblich, den Raum zwischen Spule und Membran zu evakuieren. Alternativ ist es zur Verbesserung der elektromagnetischen Kopplung von Membran zur Spule üblich, das Ausbreitungsmedium zwischen Membran und Patient mit einem Überdruck zu beaufschlagen, so daß die Membran an die Flachspule angedrückt wird.

Ein derartiger Aufbau ist jedoch mit gewissen Nachteilen behaftet. So ist der Überdruck im Ausbreitungsmedium, vor allem wenn keine zusätzliche Trennmembran eingesetzt wird, nur in einem geringen Druckbereich möglich, da das Ausbreitungsmedium, üblicherweise Wasser, bei allen modernen Stoßwellenquellen in einem gummiartigen Koppelkissen eingeschlossen ist, das wiederum nur einen geringen Überdruck zuläßt. Ferner ist für die Ausbildung und die Einhaltung des Vakuums zwischen der Flachspule und der Membran sowohl eine Vakuumpumpe als auch zusätzliche Dichtungsmaßnahmen erforderlich. Sowohl der Überdruck als auch der Unterdruck müssen elektrisch angesteuert und überwacht werden, wozu noch ein nicht unerheblicher Wartungsaufwand für die die Drücke erzeugenden Pumpen gehören.

Die DE C 4242131 beschreibt einen akustischen Druckimpulsgenerator zur Erzeugung von akustischen Druckimpulsen in einem akustischen Ausbreitungsmedium, der eine stoßartig antreibbare, an das akustische Ausbreitungsmedium angrenzende Membran aufweist, die unter mechanischer Vorspannung steht, so daß sie nach Erzeugung eines Druckimpulses in ihre Ausgangslage zurückkehrt. Der Rand der Membran ist dabei derart fixiert, daß die Membran innerhalb ihres Randes am Stoßerregungssystem anliegt, wobei sie vor der Montage zur Spule hin gewölbt ist und die Vorspannung durch Andrücken der Membran längs ihres Randes an das Stoßerregungssy;stem aufgebracht wird. Diese Membran steht somit unter Druckspannung.

Bei diesem bekannten Druckimpulsgenerator soll sichergestellt werden, daß vor der Erzeugung eines Druckimpulses ein Aufliegen der Membran auf dem Stoßerregungssystem und nach der Erzeugung eines Druckimpulses die Rückkehr der Membran in ihre Ausgangslage gewährleistet ist.

Die Membran selber ist dabei ein Verbundbauteil, das aus einer gewölbten metallischen Scheibe und einem einspannbaren Randteil besteht, die miteinander mechanisch, z. B. durch Vulkanisieren verbunden sind. Abgesehen von den enormen technischen Aufwendungen, die zur Herstellung eines derartigen Verbundbauteils notwendig sind, tritt noch der wesentliche Nachteil auf, daß eine gewölbte metallische Scheibe nur durch Auftreten stauchender axial einwirkender Kräfte plan an die Oberfläche einer Flachspule anlegbar ist, ohne Falten zu werfen.

Aufgabe der vorliegenden Erfindung ist es, eine Stoßwellenquelle zu, schaffen, die einen besonders einfachen, kostengünstigen Aufbau mit hohem Wirkungsgrad aufweist.

Ausgehend von einer elektromagnetischen Stoßwellenquelle der eingangs näher genannten Art erfolgt die Lösung dieser Aufgabe mit den im kennzeichnenden Teil des Anspruchs 1 angegebenen Merkmalen; vorteilhafte Ausgestaltungen sind in den Unteransprüchen beschrieben.

Die erfindungsgemäß ausgestaltete Stoßwellenquelle bietet nicht nur den Vorteil, daß der Einsatz von Überdruck vor der Membran und/oder von Unterdruck zwischen Membran und Flachspule überflüssig ist, so daß der gesamte damit verbundene Aufwand zur Druckeinstellung und zur Drucküberwachung entfällt, samt dem dazugehörigen Dichtungsaufwand, sondern auch den Vorteil eines besonders einfachen Aufbaus der die Stoßwellen erzeugenden Anordnung aus Membran und Flachspule. Die ebene, d. h. plane Membran benötigt kein separates Randteil, mit welchem es verklebt werden müßte, so daß die Herstellung deutlich billiger ist.

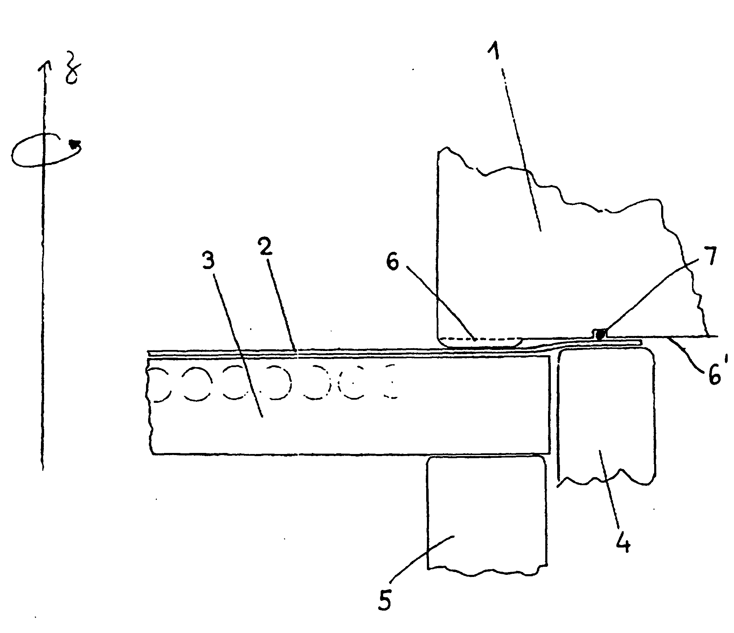

Im folgenden wird die Erfindung anhand der Zeichnung näher erläutert, in der ein vorteilhaftes Ausführungsbeispiel dargestellt ist; die einzige Figur zeigt dabei einen Schnitt durch einen Randteil einer Einspannung der Membran mit der zugehörigen Flachspule.

In der Figur ist mit 1 ein Strukturteil einer Stoßwellenquelle bezeichnet, die zur Einspannung des die Stoßwellen erzeugenden Teils eingesetzt wird, wobei letzterer aus einer Membran 2 aus einem elektrisch leitenden Material besteht, die auf ihrer einen Seite mit einem (nicht dargstellten) akustischem Ausbreitungsmedium in Berührung steht und die auf ihrer anderen Seiten über einen Isolator mit einer Flachspule 3 in Verbindung steht, wobei die Flachspule 3 aus spiralförmig angeordneten Windungen bestehen kann, die in einen entsprechenden Isolator eingebettet sind. Als zusätzlicher Isolator kommt auch eine zwischen der Flachspule 3 und der Membran 2 angeordnete Folie in Betracht.

Erfindungsgemäß steht nun die metallische Membran 2 unter einer radialen Vorspannung, die dadurch erzeugt wird, daß beim Einklemmen des Randteils der im allgemeinen kreisförmig ausgebildeten Membran 2 zwischen dem Strukturteil 1 und dem Strukturteil 4 eine entsprechende Vorspannung erzeugt wird.
Zu diesem Zweck ist die Unterseite 6' des Strukturteils 1 mit einem Wulst 6 versehen und das Strukturteil 4 derart an seiner am Strukturteil 1 anliegenden Oberfläche ausgestaltet, daß beim Anpassen der beiden Strukturteile 1, 4 aneinander z. B. durch axiales Verschrauben die Membran 2 radial nach außen gezogen wird (ähnlich dem bekannten Tiefziehverfahren); dies geschieht dadurch, daß das Strukturteil 4 den Randbereich der Membran 2 über den mit 6 bezeichneten Wulst zieht und dadurch verformt. Mit 7 ist eine Abdichtung zwischen Strukturteil 1 und Oberfläche der Membran 2 bezeichnet.

Diese radiale Einspannung der plan an der Flachspule 3 anliegenden metallischen Membran 2 bietet den großen Vorteil, daß ein dauerhaftes planes Anliegen der Membran 2 an der Flachspule 3 gewährleistet ist. Dabei kann die Membran 2 mit einer Schichtdicke von ca. 0,1 bis. 0,3 mm aus Aluminiumlegierungen bestehen, deren Zugfestigkeit größer als 250 N/mm² sind, wobei ein geeignetes Material eine Legierung aus Aluminium ist, welches 1 bis 4 % Mangan und/oder Magnesium und/oder Chrom aufweist.

Erfindungswesentlich ist also, daß die die Stoßwellen erzeugende Membran zwischen den einzelnen elektrischen Beaufschlagungen der Flachspule möglichst dicht an dieser anliegt und dabei nicht nur eben ausgestaltet ist, sondern unter einer radialen Vorspannung steht, die-jegliche Wölbung, außer während der Erzeugung der Stoßwellen, verhindert.

## Patentansprüche

1. Elektromagnetische Stoßwellenquelle, deren die Stoßwellen erzeugender Teil eine Membran (2) aus elektrisch leitendem Material ist, die auf ihrer einen Seite mit einem akustischen Ausbreitungsmedium in Berührung steht und die auf der anderen Seite über einen Isolator mit einer Flachspule (3) in Verbindung steht, wobei die Membran unter mechanischer Vorspannung steht, **dadurch gekennzeichnet, daß** die Membran eben ausgebildet ist und daß die Vorspannung durch eine parallel zur Oberfläche wirkende Anordnung (4, 6) erzeugt wird.

2. Stoßwellenquelle nach Anspruch 1, **dadurch gekennzeichnet, daß** die Anordnung zur Erzeugung der radialen Vorspannung aus zwei, den Rand der Membran einspannenden Strukturteilen besteht (4, 6), wobei das eine Strukturteil eine Aussparung aufweist (6), in die das andere Strukturteil unter Mitnahme des Randes der Membran eingreift.

3. Stoßwellenquelle nach einem der vorhergehenden Ansprüche, **dadurch gekennzeichnet, daß** die Membran aus einer Legierung besteht, deren Zugfestigkeit größer als 250 N/mm² ist.

4. Stoßwellenquelle nach Anspruch 3, **dadurch gekennzeichnet, daß** die Legierung eine 1 bis 4 % Mangan und/oder Magnesium und/oder Chrom aufweisende Aluminiumlegierung ist.

## Claims

1. Electromagnetic shockwave source, whose part which produces the shockwaves is a membrane (2) composed of electrically conductive material, one of whose sides is in contact with an acoustic propagation medium, and which is connected on the other side via an insulator to a flat coil (3), with the membrane being mechanically prestressed, **characterized in that** the membrane is planar, and **in that** the prestress is produced by an arrangement (4, 6) which acts parallel to the surface.

2. Shockwave source according to Claim 1, **characterized in that** the arrangement for producing the radial prestress comprises two structure parts (4, 6) which clamp in the edge of the membrane, with one structure part having a cutout (6) into which the other structure part engages, carrying the edge of the membrane with it.

3. Shockwave source according to one of the preceding claims, **characterized in that** the membrane is composed of an alloy whose tensile strength is greater than 250 N/mm².

4. Shockwave source according to Claim 3, **characterized in that** the alloy is an aluminium alloy with 1 to 4% of manganese and/or magnesium and/or chromium.

## Revendications

1. Source d'ondes de choc électromagnétique, dont la partie qui produit les ondes électromagnétiques est une membrane (2) en matériau conducteur d'électricité, qui est d'un côté en contact avec un support de propagation sonore et qui est de l'autre côté relié avec une bobine plate (3) par le biais d'un isolateur, la membrane se trouvant sous précontrainte mécanique, **caractérisée en ce que** la membrane est de forme plane et que la précontrainte est produite par un arrangement (4, 6) agissant parallèlement à la surface.

2. Source d'ondes de choc selon la revendication 1, **caractérisée en ce que** l'arrangement destiné à produire la précontrainte radiale se compose de deux éléments de structure (4, 6) qui enserrent le bord de la membrane, l'un des éléments de structure présentant un creux (7) dans lequel pénètre l'autre élément de structure en entraînant le bord de la membrane.

3. Source d'ondes de choc selon l'une des revendications précédentes, **caractérisée en ce que** la membrane se compose d'un alliage dont la résistance à la traction est supérieure à 250 N/mm².

4. Source d'ondes de choc selon la revendication 3, **caractérisée en ce que** l'alliage est un alliage d'aluminium présentant 1 à 4% de manganèse et/ou de magnésium et/ou de chrome.
